Europäisches Patentamt

European Patent Office

Office européen des brevets

(19)

(11) Publication number: **0 400 710**
**A1**

## (12) EUROPEAN PATENT APPLICATION

(21) Application number: **90201262.4**

(22) Date of filing: **18.05.90**

(51) Int. Cl.5: **G01N 33/569, //C07K7/08**

(30) Priority: **22.05.89 NL 8901270**

(43) Date of publication of application:
**05.12.90 Bulletin 90/49**

(84) Designated Contracting States:
**AT BE CH DE DK ES FR GB GR IT LI NL SE**

(71) Applicant: **AKZO N.V.**
**Velperweg 76**
**NL-6824 BM Arnhem(NL)**

(72) Inventor: **Schalken, Johannes Jacobus**
**De Kuiper 7**
**NL-5283 MK Boxtel(NL)**
Inventor: **Sprengers, Erik Dagobert**
**Dordognelaan 29**
**NL-5627 HB Eindhoven(NL)**
Inventor: **Hellings, Jan Albert**
**Lierenbout 24**
**NL-5283 AT Boxtel(NL)**

(74) Representative: **Hermans, Franciscus G.M. et al**
**Patent Department AKZO N.V. Pharma Division P.O. Box 20**
**NL-5340 BH Oss(NL)**

(54) **Diagnostic test for detecting antibodies directed against antigens of one or more related viruses.**

(57) The invention relates to a test for detecting antibodies in a fluid which antibodies are directed against antigens of one or more related viruses. Preferably a solid phase has been coated with a viral lysate from a group of related viruses and with a quantity of one or more antigenic peptides from other viruses from this group.

EP 0 400 710 A1

**Diagnostic test for detecting antibodies directed against antigens of one or more related viruses.**

The invention relates to a test for detecting antibodies in a fluid which antibodies are directed against antigens of one or more related viruses.

Tests known for this purpose are those in which a crude lysate of cells infected with a virus is adsorbed on a solid phase, such as a test in which a crude lysate of cells infected with HIV-1 (Human Immunodeficiency Virus) is used to detect antibodies against the HIV virus and herewith infection with this virus, causing the "Acquired Immuno Deficiency Syndrome" (AIDS), can be demonstrated.

However, this syndrome can be caused by more viruses than HIV-1. Although these viruses are related to the HIV-1 virus in such a way that antibodies directed against other HIV viruses also bind to HIV-1 antigens (preserved proteins/genes) to a certain extent, the sensitivity of tests of this type for antibodies against these other HIV viruses leaves something to be desired.

There are several diseases which can be caused by groups of related viruses. Herpes viruses, hepatitis viruses, etc. may be considered.

It is frequently important in the first instance to detect the infection with a virus from the group of related viruses, after which specific tests can be used to establish with which variant the patient is infected.

The present invention provides an initial test of this type.

According to the invention:

1) a quantity of antigen material of one virus from a group of related viruses and

2) a quantity of one or more antigenic peptides from other viruses from this group are adsorbed on a solid phase.

Surprisingly, with a test of this type, the sensitivity for antibodies against the antigen material of the one virus is retained to a considerable degree, while the sensitivity for the other virus (or the other viruses) is appreciably improved.

It does not require any further explanation that other methods of adhesion to the solid phase than only adsorption are also possible.

For the present invention, antigen material denotes, for example, recombinant protein from a virus, or a protein fraction purified from infected cells (i.e. : a preparation obtained via known separation techniques which contains one or more viral proteins/antigens).

Preferably, a viral lysate is adsorbed as antigen material on the solid phase. The use of a lysate of this type has the advantage that, in principle, all antigenic determinants of the virus are present. As a result, the preserved proteins which are recognized by antibodies directed against related viruses are thus also present.

However, using purified proteins for example p24 or gp160 of HIV-1 as antigen material on the solid phase surprisingly yields a test with very good sensitivity.

Antigenic peptides are understood to be synthetic peptides which are analogous to parts of proteins which comprise epitopes of one or more viruses which are related to the virus from which the antigen material is derived. Said peptides can be prepared by recombinant DNA technology or by organic chemical synthesis, for instance the Merrifield-synthesis.

These synthetic peptides are coupled (conjugated) to carrier material. Carrier material can be any suitable carrier material, such as polymers or an inert (not antigenic) protein, for example BSA. The ratio between carrier material and peptide can vary between 1:50 and 1:1 (mole/mole).

According to the invention a very good AIDS test can be obtained when a HIV-1 lysate is adsorbed on a solid phase in combination with a synthetic peptide from another virus which causes AIDS. Instead of HIV-1 lysate one can also use purified proteins of HIV-1 especially p24 and gp160 of HIV-1 as mentioned above.

A preferred embodiment of such a test consists in that a HIV-1 lysate and a synthetic peptide which contains an epitope of the HIV-2 virus (conjugated to carrier material) are adsorbed on a solid phase.

A peptide which can be used for this purpose is, for example, a peptide which contains Leu-Asn-Ser-Trp-Gly-Cys-Ala-Phe-Arg-Gln-Val-Cys-, described in Science (vol. 237, 11-9-87).

A good sensitivity for several related viruses is obtained when the following are adsorbed successively on the solid phase:

a) a quantity of antigen material,

b) successively, or in one step, one or more antigenic peptides conjugated to carrier material.

The greatest sensitivity for several related viruses is obtained when the following are adsorbed successively on the solid phase:

a) one or more antigenic peptides conjugated to carrier material

b) successively, or in one step, a quantity of antigen material.

If desired, an inert protein can then be adsorbed on the solid phase. This is done in order to prevent any aspecific reactions. With a test prepared in this way, the sensitivity of the test for the first virus (the antigen material) is retained virtually in its entirety, while the sensitivity for the second (and if desired also a third and/or fourth) virus is clearly improved.

The quantities of antigen material and synthetic peptides which adhere to the solid phase are not critical. Good results are obtained when a solution of 2-10 $\mu$g protein/ml of the antigen material and a solution of 1-20 $\mu$g protein/ml of the conjugates are adsorbed successively overnight at 4°C. In order to preclude aspecific reactions, the test material is then also incubated at ambient temperature with a solution of an inert protein, for example a 0.2% solution of BSA. The fluid is then separated from the solid phase and the solid phase is dried. Many materials can be chosen as the solid phase for the present test. This choice is dependent on the embodiment which is chosen for the test. Test kits can consist of test strips of, for example, cellulose derivatives, glass fibres or other nonwovens and many polymers. Those skilled in the art can choose a suitable material depending on the embodiment of the test. A so-called Micro-ELISA plate can also be chosen, for example.

The detection of the antibody to be revealed can be carried out in various ways. The simplest is a "sandwich immunoassay", in which, after incubation with the fluid and a possible washing step, an incubation with a labelled antibody directed against the first antibody to be detected (or the first antibodies) follows. Instead of an incubation with a labelled antibody one can easily perform an incubation with a labelled antigen. For instance horse-radish peroxidase coupled to p24 and/or gp160 of HIV-1 and/or a (synthetic) peptide of HIV-2. All further known labels, such as the mentioned enzyme, other enzymes, metal sols, coloured latex sols, fluorescent compounds, chemiluminescent compounds, radionuclides, pigments, dye stuff sols, can be chosen as label. Of these labels, the direct labels (labels which require no further treatment) are to be preferred because of the simplicity.

The labels can be coupled to the antibodies or antigens by all known methods.

The invention will be illustrated with the aid of the following examples.

<u>Example 1</u>

The method.

a) Preparation of peptide BSA conjugates.

Peptide BSA conjugates are made in accordance with standard techniques such as glutaraldehyde coupling, carbodiimide coupling, disulphide coupling and thioether coupling.

For example: 1 ml of a solution of BSA (20 mg/ml) in 0.1 M NaPO$_4$ buffer (pH = 7.0) is mixed with 1 ml of peptide solution (1 mg/ml)in the same buffer. 1 ml of a 21 mM glutaraldehyde solution in 0.1 M NaPO$_4$ buffer (pH = 7.0) is added to this mixture dropwise, with shaking. The resulting mixture is incubated overnight at ambient temperature in the dark on a roller bench.

It is then dialysed for 24 hours against a 0.1 M NaPO$_4$ buffer (pH = 7.0) in order to remove the excess glutaraldehyde. The preparations are stored at -20°C until they are used.

b) Adsorption on Micro-ELISA plates.

Micro-ELISA plates are incubated overnight at 4°C with a mixture of a HIV-1 viral lysate and a BSA-HIV-2 peptide conjugate in approximately equal amounts to give a final concentration of 2-10 $\mu$g protein/ml in a 0.05 M NaHCO$_3$ buffer (pH = 9.6). The plates are then emptied and filled with a 0.2% solution of BSA. The plates are incubated with this solution for between 10 and 40 minutes at ambient temperature. The plates are then emptied and dried.

c) Sequentially adsorbed Micro-ELISA plates.

The same preparation method as under b) is followed except that the mixture is not adsorbed but, successively, a solution of viral lysate of the same final concentration as the mixture under b) and one or more solutions of synthetic peptides of final concentrations of between 1 and 20 μg protein/ml are adsorbed on the solid phase.

d) Immunoassays

Serum is diluted 1:34 with sample diluent. 100 μl is pipetted into a well of the treated Micro-ELISA plates. The plates are incubated at 37°C for 30 minutes. The plates are then emptied.

e) Detection

Directly after the incubation with serum, the plates are washed four times with a phosphate-buffered physiological saline solution to which 0.05% Tween 20 (PBS-T) has been added.

The plates are then incubated with an antibody (goat antihuman immunoglobulin) labelled with horseradish peroxidase (HRP) in a concentration of 1-10 μg/ml. The plates are then washed four times with PBS-T and incubated with a substrate solution (urea peroxide 0.5 mg/ml, orthophenyldiamine 1.6 mg/ml). The quantity of bound HRP is measured by measuring the absorption of light at 492 nm. The result is quantified by means of a "Micro-ELISA reader".

The results of the experiments are shown in Table I and Table II.

In Table I and II the B numbers such as B238798-7 indicate different sera containing antibodies. NHS stands for normal human serum.

The figures 1:10, 1:100 etc indicate the pre-dilution of serum with normal human serum. The samples are then, as already mentioned above, also diluted with sample diluent (1:34). Sample diluent contains 20% goat serum (PBS) and 1% Triton X-100.

The HIV-2 synthetic peptide is a peptide consisting of the sequence mentioned above.

ND indicates an experiment not carried out.

SIV = Simian Immunodeficiency Virus

Table I

| Sera: | HIV-1 viral lysate (2 μg/ml) | HIV-2 viral lysate (3 μg/ml) | HIV-2 peptide BSA (3 μg/ml) | HIV-1 viral lysate (2 μg/ml) mixed with HIV-2 viral lysate (3 μg/ml) | HIV-1 viral lysate (2 μg/ml) mixed with HIV-2 synthetic peptide-BSA conjugate (3 μg/ml) |
|---|---|---|---|---|---|
| anti-HIV-2 B238798-7 1:10 | 0.159 | 1.15 | 0.495 | 0.656 | 0.427 |
| -8 | 0.135 | 0.929 | 1.03 | 0.572 | 0.871 |
| -14 | 0.698 | 1.01 | 0.212 | 1.01 | 0.916 |
| -15 | 0.271 | 1.76 | 1.08 | 1.19 | 0.836 |
| -16 | 0.142 | 1.33 | 1.60 | 0.747 | 1.16 |
| B238798-7 1:100 | 0.099 | 0.224 | 0.135 | 0.188 | 0.156 |
| -8 | 0.098 | 0.204 | 0.186 | 0.176 | 0.188 |
| -14 | 0.136 | 0.208 | 0.124 | 0.219 | 0.197 |
| -15 | 0.126 | 0.405 | 0.201 | 0.304 | 0.198 |
| -16 | 0.110 | 0.305 | 0.301 | 0.237 | 0.285 |
| anti-SIV-F1 B249341 | 0.078 | 0.445 | 1.78 | 0.242 | 1.04 |
| anti-HIV-1 B180811 1:10 | 2.13 | 0.156 | 0.087 | 1.36 | 1.92 |
| 1:30 | 1.66 | 0.104 | 0.085 | 0.865 | 1.23 |
| 1:100 | 0.939 | 0.094 | 0.086 | 0.460 | 0.601 |
| 1:300 | 0.436 | 0.091 | 0.080 | 0.222 | 0.322 |
| 1:800 | 0.218 | 0.092 | 0.090 | 0.137 | 0.167 |

"Mixtures of HIV-1 and HIV-2 antigens adsorbed on Micro-ELISA plates. Absorption at 492 nm."

It can be seen from Table I that the sensitivity of a test containing exclusively HIV-1 viral lysate for HIV-2 and SIV sera is low. A mixture of HIV-1 lysate and HIV-2 lysate has a good sensitivity for HIV-2 serum, but the sensitivity for HIV-1 serum is clearly lower. The sensitivity for SIV serum is very low. A high sensitivity for all three sera is obtained with a mixture of HIV-2 peptide/BSA and HIV-1 viral lysate.

Tabel IIa

| | A492 nm after 30 min. | | | |
|---|---|---|---|---|
| sera: | HIV-2 synthetic peptide | HIV-2 synthetic peptide BSA-Conjugate (A492 after 15 min.) | HIV-1 viral lysate | HIV-1 viral lysate + second layer HIV-2 peptide BSA conjugate |
| Sample diluent | 0.076 | 0.072 | 0.084 | 0.084 |
| NHS 238763-pool | 0.164 | 0.404 | 0.258 | 0.363 |
| -8 | ND | ND | 0.170 | 0.244 |
| -10 | 0.189 | 0.348 | 0.189 | 0.270 |
| -11 | 0.130 | 0.205 | 0.172 | 0.222 |
| -14 | 0.167 | ND | 0.159 | 0.176 |
| -15 | ND | ND | 0.187 | 0.270 |
| anti-HIV-1 pool B 180811 | 0.588 | 0.453 | 2.55 | 2.81 |
| anti-SIV F1 B249341 | 1.148 | 1.855 | 0.175 | 1.14 |

Tabel IIb

| | A492 nm after 5 min. | | | |
|---|---|---|---|---|
| sera: | HIV-2 synthetic peptide | HIV-2 synthetic peptide BSA-conjugate A492 15 min. | HIV-1 viral lysate | HIV-1 viral lysate + second layer HIV-2 peptide BSA conjugate |
| NHS 238763-pool | 0.084 | 0.139 | 0.100 | 0.112 |
| anti-HIV-2 pool B 238798-7 | 0.747 | 1.353 | 0.301 | 0.875 |
| -8 | 0.730 | 1.51 | 0.203 | 0.971 |
| -14 | 0.286 | 0.910 | 0.758 | 1.04 |
| -15 | 0.653 | 1.29 | 0.717 | 1.131 |
| -16 | 1.12 | 1.54 | 0.381 | 1.07 |

6

Table III

| Comparison of adsorbed mixture and sequentially adsorbed Micro-ELISA plates. | | | |
|---|---|---|---|
| sera | HIV-1 viral lysate 2μg/ml | Mixture of viral lysate 2 μg/ml and BSA-HIV-2-peptide 2 μg/ml | Viral lysate and BSA-HIV-2-peptide sequentially adsorbed |
| anti-HIV-2 B238798-7 1:25 | 0.221/0.199 | 0.473 | 0.287 |
| -8 | 0.195/0.247 | 0.842 | 0.392 |
| -14 | 1.01/1.48 | 0.827 | 1.23 |
| -15 | 0.558/0.600 | 0.987 | 0.713 |
| -16 | 0.214/0.252 | 1.09 | 0.515 |
| anti-SIV-F1 B249341 | 0.113/0.093 | 0.405 | 0.344 |
| anti-HIV-1 B180811-1 1:10 | 2.52/2.64 | 2.52 | 2.70 |
| 1:30 | 2.47/2.57 | 2.14 | 2.58 |
| 1:100 | 2.08/1.87 | 1.26 | 2.05 |
| 1:300 | 1.28/1.16 | 0.831 | 1.16 |
| 1:800 | 0.654/0.557 | 0.447 | 0.599 |
| NHS B249969-16 | 0.086/0.088 | 0.112 | 0.084 |
| -17 | 0.087/0.092 | 0.092 | 0.099 |
| -18 | 0.251/0.253 | 0.242 | 0.313 |
| -19 | 0.297/0.349 | 0.404 | 0.348 |
| -20 | 0.086/0.097 | 0.104 | 0.104 |

It can be seen from these three tables that aspecific binding is not excessively increased by choosing the method of sequential adsorption of HIV-1 lysate and HIV-2 synthetic peptide-BSA conjugate.

In addition, the tables show that the sensitivity of the test thus obtained is clearly the highest for the various antisera.

Example 2

One-step sandwich assay using antigen-HRP conjugates

a) coating

A mixture of HIV-1 antigens (p24 and gp160) and a BSA-HIV-2 peptide conjugate are incubated in a microwell of a microtiter plate overnight at 4 °C in an 0.05 M NaHCO₃ buffer solution, pH 9.6 in approximately equal amounts. Subsequently the plates were emptied and washed sequentially with Tris-NaCl-Tween 20 buffer (0.04 M, 0.15 M, 0.05% Tween 20), a Tris-NaCl buffer (0.04 M, 0.15 M) and 2x with a 0.04 M Tris buffer. The plates are then emptied and dried.

b) immuno assays

Serum is pipetted undiluted in a coated microwell (100 μl). Furthermore 50 μl conjugate mixture is added containing HRP conjugate of HIV-1 p24, HIV-1 gp160 and HIV-2-peptide. Subsequently an incubation of 60 min. has been performed.

c) Detection

Directly after the incubation with serum, the plates were washed four times with a phosphate-buffered physiological saline solution to which 0.05% Tween 20 (PBS-T) has been added. After 30 min. the reaction is stopped with 1 M $H_2SO_4$ and an extinction at 492 nm has been measured.

Table IV

| Analytical sensitivity for anti-HIV-1 and anti-HIV-2 in immuno assays using goat-antihuman HRP conjugate (two step assay) or using antigen-HRP conjugate (one step assay) respectively. | | |
|---|---|---|
| sera | Analytical sensitivity * | |
| | two step assay GaH-HRP (goat antihuman) | one step assay antigen-HRP |
| aHIV-1 | | |
| B 180811 | 6.400 | 30.000 |
| B 115474-10 | 12.800 | 100.000 |
| B 203572-11 | 12.800 | 100.000 |
| B 203572-16 | 12.800 | 30.000 |
| B 203572-4 | 8.000 | 30.000 |
| aHIV-2 | | |
| B 265412-3 | 400 | 3.000 |
| -7 | 800 | 3.000 |
| -9 | 800 | 3.000 |
| pool 433 | 100 | 1.000 |

* reciprocal dilution of an anti-HIV serum (in normal human serum), which still reacts positively in the corresponding test.

One can easily see from Table IV that the one-step assay has a superior analytical sensitivity for both anti-HIV-1 and anti-HIV-2.

**Claims**

1. Test for detecting antibodies in a fluid which antibodies are directed against antigens of one or more related viruses, characterized in that :
    1) a quantity of antigen material of one virus from a group of related viruses and
    2) a quantity of one or more antigenic peptides from other viruses from this group are adsorbed on a solid phase.
2. Test according to Claim 1, characterized in that the antigen material is a viral lysate.
3. Test according to Claim 2, characterized in that the viral lysate is HIV-1 lysate.
4. Test according to one of the preceding claims, characterized in that one of the antigenic peptides is a peptide which contains a HIV-2 epitope.
5. Test according to Claim 4, characterized in that the HIV-2 epitope contains the sequence Leu-Asn-Ser-Trp-Gly-Cys-Ala-Phe-Arg-Gln-Val-Cys.
6. Test according to one of the preceding claims, characterized in that BSA is the carrier material to which the antigenic peptides are coupled.
7. Method for the preparation of a test according to one of the preceding claims, characterized in that the following are adsorbed successively on a solid phase:
    a) a quantity of antigen material and
    b) successively, or in one step, one or more antigenic peptides.

8. Method for the preparation of a test according to one of the claims 1-6, characterized in that the following are adsorbed successively on a solid phase:

    a) one or more antigenic peptides

    b) successively, or in one step, a quantity of antigen material.

9. Method for detecting a virus from a group of related viruses, characterized in that a test according to one of Claims 1 to 6 is incubated with the fluid to be tested, after which detection is carried out by means of a method known per se.

10.Test kit for carrying out the method according to Claim 9.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 349 037 (AKZO N.V.)<br>* See whole article * | 1-10 | G 01 N 33/569 //<br>C 07 K 7/08 |
| Y | WO-A-8 901 527 (CELLULAR PRODUCTS INC.)<br>* See abstract; pages 15-18; conclusions 1,6 * | 1-10 | |
| Y | EP-A-0 292 454 (VIROVAHL S.A.)<br>* See the whole document * | 1-10 | |
| Y | INNOGENETICS, BIOTECHNOLOGY FOR THE HEALTH CARE FIELD, April 1989, Antwerpen, BE<br>* See the whole document * | 1-10 | |
| Y | EP-A-0 284 383 (GENETIC SYSTEMS COR.)<br>* See the whole document * | 1-10 | |
| Y | BRITISH MEDICAL JOURNAL, vol. 296, 9th January 1988, pages 83-86, London, GB; D.C.W. MABEY et al.: "Human retroviral infections in The Gambia: prevalence and clinical features"<br>* See the whole article * | 1-10 | TECHNICAL FIELDS SEARCHED (Int. Cl.5)<br><br>G 01 N |
| Y | SCIENCE, vol. 237, 11th September 1987, pages 1346-1349; J.W. GNANN, Jr. et al.: "Synthetic peptide immunoassay distinguishes HIV type I and HIV type 2 infections"<br>* See the whole article * | 1-10 | |
| Y | WO-A-8 504 903 (UNITED STATES OF AMERICA)<br>* See conclusions 26-30 * | 1 | |

The present search report has been drawn up for all claims

| Place of search | Date of completion of the search | Examiner |
|---|---|---|
| THE HAGUE | 29-08-1990 | OSBORNE H.H. |